# EUROPÄISCHE PATENTSCHRIFT

(11) **EP 0 686 392 B1**
(45) Veröffentlichungstag und Bekanntmachung des Hinweises auf die Patenterteilung: **02.12.1998**
(21) Anmeldenummer: 95107499.6
(22) Anmeldetag: 17.05.1995
(51) Int. Cl.: A61K 9/16, A61K 9/20, A61K 31/00, A61K 31/19

(54) **Thermisches Granulierverfahren**
Thermal granulating process
Procédé thermique de granulation

(30) Priorität: 30.05.1994 DE 4418837
(43) Veröffentlichungstag der Anmeldung: 13.12.1995
(73) Patentinhaber: BAYER AG, 51368 Leverkusen (DE)
(72) Erfinder: Hürner, Ingrid, D-40479 Düsseldorf (DE); Danz, Peter, D.I., D-50996 Köln (DE); Walter, Reinhard, Dr., D-51375 Leverkusen (DE); Maasz, Joachim, Dr., Granger, IN 46530 (US); Frank, Georg, Dr., D-42115 Wuppertal (DE)

(56) Entgegenhaltungen:
- WO-A-92/06679
- DE-A- 3 833 448
- DE-A- 4 031 881
- US-A- 5 169 645

## Beschreibung

Die Erfindung betrifft ein thermisches Verfahren zur Herstellung eines direkt tablettierbaren Granulates und daraus hergestellte Arzneizubereitung.

Zur industriellen Herstellung von Tabletten werden Direktttablettierungsverfahren bevorzugt. Direkttablettierung bedeutet, daß die Inhaltsstoffe einer Tablette direkt verpreßt werden ohne vorausgehende Granulationsprozesse. Hierdurch entfallen zeit- und kostenaufwendige Verfahrensschritte, wie z.B. Lösungsmittelentfernung und Trocknung nach einer Feuchtgranulation.

An Pulvermischungen, die direkt tablettiert werden sollen, werden jedoch hohe Anforderungen bezüglich Fließfähigkeit, Dichte, Partikelgröße etc. ihrer Einsatzstoffe gestellt, um eine gleichmäßige, homogene Zuführung des Pulvers zur Tablette und damit eine Chargenhomogenität zu gewährleisten. Viele Wirkstoffe, wie z.B. Ibuprofen und Ketoprofen sind durch ihre ungünstigen physikalischchemischen Eigenschaften für eine Direkttablettierung nicht geeignet.

Für niedrig schmelzende Wirkstoffe, d.h. für Wirkstoffe mit einem Schmelzpunkt zwischen 30 und 200°C, vorzugsweise zwischen 40 und 150°C, wurde nun ein Verfahren gefunden, den Wirkstoff direkt zusammen mit den gewünschten üblichen Hilfsstoffen in einfacher Weise zu einem direkt tablettierbaren Granulat zu verarbeiten.

Aus dem Stand der Technik sind bereits Verfahren bekannt, wonach Tablettiergut thermisch behandelt wird, als Vorbereitung zur einer Direkttablettierung. Die deutsche Offenlegungsschrift DE 3 833 448 beschreibt ein Verfahren zur Gewinnung von Ibuprofenzubereitungen durch Direkttablettierung. Nach diesem Verfahren wird der Wirkstoff in einer Schmelzapparatur komplett aufgeschmolzen und dann an einem Kontaktkühlapparat verfestigt und kristallisiert. Die entstehenden Wirkstoffschuppen werden zu einem Pulver mit verbesserten Fließeigensehaften vermahlen. Dieses Ibuprofen-Pulver kann mit geeigneten Hilfsstoffen vermischt und dann tablettiert werden.

In WO 92/06679 wird ein Verfahren beschrieben nach welchem der Binder bei Temperaturen die immer unterhalb des Schmelzpunktes des Wirkstoffs gehalten werden sollen, aufgeschmolzen wird. Als Wirkstoffe werden nur solche mit einem Schmelzpunkt über 120° eingesetzt, die bei den verwendeten Temperaturen von max. 100° C immer unverändert bleiben.

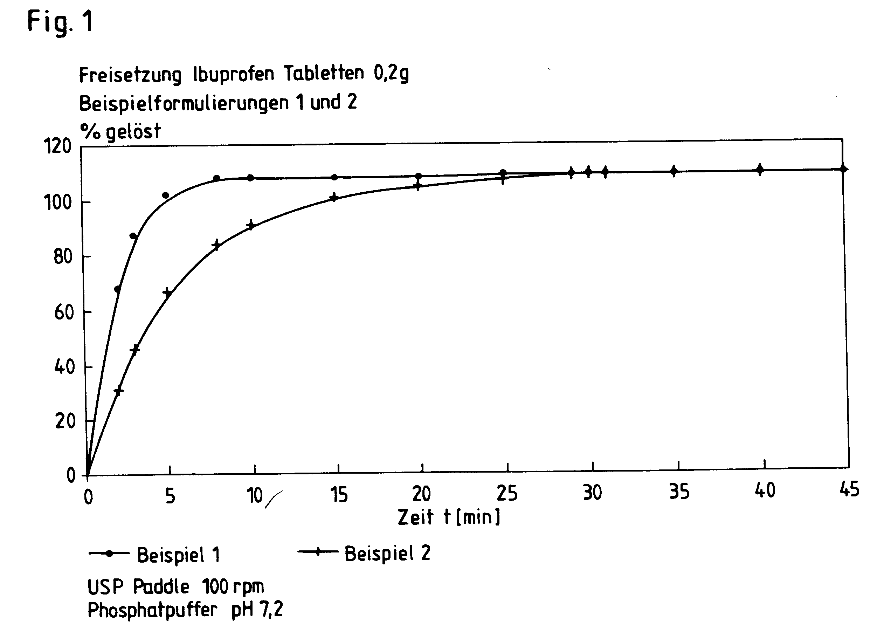

In DE-4 031 881 wird ein Verfahren zur Herstellung von Formulierungen mit verzögerter Wirkstofffreisetzung beschrieben. Hierzu werden matrixbildende Hilfsstoffe, deren Menge deutlich über der Menge des eingesetzten Wirkstoffs liegt, zur Erreichung der Retardierung eingesetzt. Hiervon unterscheidet sich das Erfindungsgemäßverfahren nach welchem zumindest ein Teil des niedrig schmelzenden Wirkstoffs aufgeschmolzen wird, eindeutig. Gleichzeitig unterscheiden sich die Freisetzungen der dort beschriebenen Retardzubereitungen eindeutig von den Freisetzungen der erfindungsgemäß hergestellten Wirkstoffe wie aus Fig. 1 ersichtlich ist.

EP-A 305 356 beschreibt ein Granulat, das durch Schmelzgranulation einer niedrig schmelzenden Substanz mit ihrer eigenen Schmelze entsteht. Durch den Schmelzüberzug auf der kristallinen Substanz werden die physikalisch-chemischen Eigenschaften, insbesondere die Fließeigenschaften positiv verändert, was zu einer leichteren Tablettierung führt.

Mit den bisher bekannten Verfahren werden jedoch nur einzelne (Wirk)stoffe für eine Direkttablettierung vorbereitet, wobei die weiteren Inhaltsstoffe der Arzneiformen noch beigemischt werden müssen. Je nach Art und Menge dieser Beimischungen können wieder die oben erwähnten pulvertechnischen Probleme auftreten die eine Direkttablettierung verhindern oder beeinträchtigen.

Eine weiterer Vorteil des erfindungsgemäßen Verfahrens liegt in der Tatsache, daß vor allem bei relativ hoch zu dosierenden Wirkstoffen wie zum Beispiel Ibuprofen nur eine geringe Menge von Hilfsstoffen zugesetzt werden muß. Dies führt zu kleinen, für den Konsumenten besonders angenehm einzunehmenden Arzneiformen.

Die Erfindung betrifft ein thermisches Verfahren zur Herstellung eines direkt tablettierbaren Granulats, enthaltend einen Wirkstoff mit Schmelzpunkt zwischen 30 und 200°C und gleichzeitig die notwendigen Tablettenhilfsstoffe, welches dadurch gekennzeichnet ist, daß ein Gemisch aus Wirkstoff und den notwendigen Hilfsstoffen mittels einer Schmelzextrusion bei Temperaturen zwischen 30 und 200°Czu einem homogenen nicht verklebenden Extrudat verarbeitet wird, das anschließend zu einem tablettierbaren Granulat zerkleinert wird.

Die Erfindung betrifft auch so hergestellte Granulate und Extrudate und deren Verwendung bei der Herstellung von Tabletten.

Bei der Herstellung dieser Granulate bzw. Extrudate erfüllen die eingesetzten Wirkstoffe mit niedrigem Schmelzpunkt die Funktion eines Bindemittels bzw. eines festen Lösungsmittels. Die so erhaltenen Granulate enthalten vorzugseise 25 bis 95 Gew.-%, vorzugsweise 35 bis 90 Gew.-% niedrig schmelzende Bestandteile aus Wirkstoff und Hilfsstoffen. Als niedrig schmelzende Wirkstoffe seien vorzugsweise aktive Substanzen mit einem Schmelzpunkt zwischen 30 und 200°C, insbesondere zwischen 40 und 150°C genannt. Hierzu gehören insbesondere nichtsteroidale analgetische Substanzen und antiinflammatorische Substanzen vom Typ der Arylpropionensäuren. Von besonderem Interesse sind die Wirkstoffe Ibuprofen, Ketoprofen, Naproxen und Indomethacin in Form ihrer Racemate und auch als Enantiomere.

Als Hilfsstoffe seien die üblichen Tablettenhilfsstoffe wie Bindemittel, Füllmittel, Zerfallshilfsmittel etc. genannt. Das erfindungsgemäß hergestellte Granulat eignet sich zur Herstellung von Arzneizbereitungen wie Tabletten, Brausetabletten, Kapseln, Trockensäften und Trockensuspensionen.

Die Herstellung erfolgt, indem der niedrig schmelzende Wirkstoff in einem Mischer mit den geeigneten Hilfsstoffen trocken gemischt und diese Mischung direkt einem beheizbaren Extruder zugeführt wird. Durch die Misch- und Knetelemente des Extruders wird die Mischung bei einer Temperatur, bei der ein Teil des Wirkstoffs aufgeschmolzen wird, zu einem Extrudat kompaktiert. Dieses Extrudat wird durch eine Lochplatte zu dünnen Strängen von 0,3 - 2,0 mm Durchmesser gepreßt und nach dem Erkalten auf die gewünschte Korngröße des Granulats zerkleinert. Die so erhaltenen Granulate können sofort der Tablettierung zugeführt werden, wobei nur noch ein Schmiermittel benötigt wird. Durch dieses erfindungsgemäße Verfahren können außer dem Schmiermittel alle Hilfsstoffe wie Bindemittel, Zerfallshilfsstoffe, Füllstoffe und sonstige Hilfsstoffe direkt in das Granulat eingearbeitet werden. Dadurch entfallen langwierige Nachverarbeitungsprozesse. Da ebenso zeitaufwendige und umweltbelastende Granulations- und Trocknungsprozesse entfallen, zeichnet sich das erfindungsgemäße Verfahren durch eine hohe Kosteneffizienz aus. Überraschenderweise zeichnen sich die erfindungsgemäß hergestellten Ibuprofen-Tabletten neben ihrer guten Tablettierbarkeit auch durch eine hervorragende Freisetzung des Wirkstoffs aus wie aus Fig. 1 ersichtlich ist. Als geeignete Hilfsstoffe die direkt nach dem erfindungsgemäßen Verfahren in die Granulate eingearbeitet werden können seien genannt: Mikrokristalline und mikrofeine Cellulose (Avicel und Elcema-Typen), Stärke und modifizierte Stärken wie Reisstärke oder Maisstärke, hochdisperses Siliciumdioxid wie Aerosil, Polyvinylpyrrolidone sowie Calciumsalze wie z.B. Calciummono-, Di- oder Triphosphate oder Calciumcarbonat, als Füllmittel, Zuckeralkohole wie Mannitol, Xylitol oder Sorbitiol) sowie als Aktivsprengmittel quervernetzte Stärke, quervernetzte Cellulose und quervernetztes Polyvinylpyrrolidon sowie andere zur Tablettierung übliche Substanzen.

### Ausführungsbeispiele

### Beispiel 1

| | Substanz | Masse [g] |
|---|---|---|
| 1. | (R,S) Ibuprofen | 2 000 |
| 2. | Avicel | 430 |
| 3. | Aerosil | 100 |
| 4. | Maisstärke | 390 |
| 5. | Ac-Di-Sol | 60 |
| 6. | Magnesiumstearat | 20 |

Die Substanzen 1 bis 5 werden in einem Taumelmischer gemischt und dann einem beheizbaren Zweiwellen-Extruder zugeführt.

Die Extrusionstemperatur beträgt 67°C.

Aus der Düsenplatte treten homogene, nicht verklebende an der Luft abkühlende Stränge aus. Diese werden dann über ein Frewitt-Sieb 0,8 mm zwangsgesiebt, so daß Granulate mit einer Korngröße zwischen 0,05 bis 0,8 mm entstehen. Die Granulate werden in einem Taumelmischer mit dem Schmiermittel Magnesiumstearat gemischt und dann direkt auf einer geeigneten Tablettenpresse zu Tabletten von 300 mg verpresst.

### Beispiel 2

| | Substanz | Masse [g] |
|---|---|---|
| 1. | (R,S) Ibuprofen | 2 000 |
| 2. | Avicel | 430 |
| 3. | Aerosil | 100 |
| 4. | Maisstärke | 450 |
| 5. | Magnesiumstearat | 20 |

Die Extrusion der Substanzen 1 bis 4 erfolgt analog dem Beispiel 1, die Extrusionstemperatur beträgt ebenso 67°C.

Die Weiterverarbeitung des Extrudates zu Tabletten mit einem Gedicht von 300 mg erfolgt wie in Beispiel 1.

Die Freisetzungscharakteristik der Beispielformulierungen 1 und 2 ist in Fig. 1 wiedergegeben.

### Beispiel 3

| | Substanz | Masse [g] |
|---|---|---|
| 1. | (R,S) Ketoprofen | 500 |
| 2. | Avicel | 500 |
| 3. | Maisstärke | 480 |
| 4. | Magnesiumstearat | 20 |

1, 2, 3 werden wie in Beispiel 1 angegeben gemischt und extrudiert, das zerkleinerte Extrudat mit dem Schmiermittel gemischt und zu Tabletten mit einem Sollgewicht von 150 mg verpresst.

Die Extrusionstemperatur beträgt 86°C.

## Patentansprüche

1. Verfahren zur Herstellung eines direkt tablettierbaren Granulates, enthaltend einen Wirkstoff mit Schmelzpunkt zwischen 30 und 200°C sowie alle notwendigen Tablettenhilfsstoffe, dadurch gekennzeichnet, daß man das Gemisch aus Wirkstoff und den Hilfsstoffen direkt mittels einer Schmelzextrusion bei Temperaturen zwischen 30 und 200°C unter teilweisem Aufschmelzen des Wirkstoffs zu einem homogenen nicht verklebenden Extrudat verarbeitet, das im Anschluß zu einem direkt tablettierbaren Granulat zerkleinert wird.

2. Verfahren gemäß Anspruch 1, dadurch gekennzeichnet, daß das eingesetzte Gemisch aus Wirkstoff und Hilfsstoffen 20 bis 95 % niedrig schmelzende Bestandteile enthält.

3. Verfahren gemäß Anspruch 1, dadurch gekennzeichnet, daß man Wirkstoffe mit einem Schmelzpunkt zwischen 40 und 150°C verwendet.

4. Verfahren gemäß Anspruch 1, dadurch gekennzeichnet, daß man als Wirkstoff nicht-steroidale analgetische Wirkstoffe und/oder antiinflammatorische Substanzen vom Typ der Arylpropionsäuren einsetzt.

5. Verfahren gemäß Anspruch 1, dadurch gekennzeichnet, daß man als Wilkstoff Ibuprofen, Ketoprofen, Naproxen oder Indomethacin als Racemate oder in ihrer enantiomerenreinen Form einsetzt.

6. Verfahren gemäß Anspruch 1, dadurch gekennzeichnet, daß man die (teilweise) aufgeschmolzene Mischung aus dem beheizbaren Extruder durch eine Lochplatte zu dünnen Strängen von 0,3 bis 2,0 mm Durchmesser preßt und diese nach dem Erkalten zu einem Granulat zerkleinert.

7. Direkt tablettierbares Granulat hergestellt gemäß Anspruch 1.

8. Verwendung des gemäß Anspruch 1 hergestellten Granulats zur Herstellung von Arzneizubereitungen.

## Claims

1. Process for the production of directly tablettable granules, containing an active compound having a melting point of between 30 and 200°C and all the necessary tablet auxiliaries, characterized in that the mixture of active compound and the auxiliaries is processed directly by means of a melt extrusion at temperatures of between 30 and 200°C with partial melting of the active compound to give a homogeneous non-agglutinating extrudate which is subsequently comminuted to give directly tablettable granules.

2. Process according to Claim 1, characterized in that the mixture of active compound and auxiliaries employed contains 20 to 95% of low-melting constituents.

3. Process according to Claim 1, characterized in that active compounds having a melting point of between 40 and 150°C are used.

4. Process according to Claim 1, characterized in that, as active compound, non-steroidal analgesic active compounds and/or antiinflammatory substances of the arylpropionic acid type are employed.

5. Process according to Claim 1, characterized in that, as active compound, ibuprofen, ketoprofen, naproxen or indomethacin are employed as racemates or in their enantiomerically pure form.

6. Process according to Claim 1, characterized in that the (partly) molten mixture from the heatable extruder is pressed through a perforated plate to give thin strands of 0.3 to 2.0 mm diameter and these are comminuted after cooling to give granules.

7. Directly tablettable granules prepared according to Claim 1.

8. Use of the granules prepared according to Claim 1 for the production of pharmaceutical preparations.

## Revendications

1. Procédé de production d'un granulé directement transformable en comprimés, contenant une substance active de point de fusion compris entre 30 et 200°C ainsi que toutes les substances auxiliaires nécessaires pour la formation de comprimés, caractérisé en ce qu'on met en oeuvre le mélange de substance active et des substances auxiliaires directement par extrusion à l'état fondu à des températures comprises entre 30 et 200°C avec fusion partielle de la substance active pour former un corps extrudé homogène non collant, qu'on fragmente ensuite en un granulé directement transformable en comprimés.

2. Procédé suivant la revendication 1, caractérisé en ce que le mélange utilisé de substance active et de substances auxiliaires contient 20 à 95 % de composants de bas point de fusion.

3. Procédé suivant la revendication 1, caractérisé en ce qu'on utilise des substances actives ayant un point de fusion compris entre 40 et 150°C.

4. Procédé suivant la revendication 1, caractérisé en ce qu'on utilise comme substance active des substances actives analgésiques et/ou des substances anti-inflammatoires non stéroïdiennes du type d'acides arylpropionique.

5. Procédé suivant la revendication 1, caractérisé en ce qu'on utilise comme substance active de l'ibuprofène, du kétoprofène, du naproxène ou de l'indométhacine à l'état de racémates ou sous leur forme énantiomère pure.

6. Procédé suivant la revendication 1, caractérisé en ce qu'on presse le mélange (partiellement) fondu, à sa sortie de l'extrudeuse chauffante, à travers une plaque perforée en minces boudins de 0,3 à 2,0 mm de diamètre et après refroidissement, on fragmente ces derniers en un granulé.

7. Granulé directement transformable en comprimés, produit conformément à la revendication 1.

8. Utilisation du granulé obtenu conformément à la revendication 1 pour la production de préparations pharmaceutiques.
